# EUROPEAN PATENT SPECIFICATION

(11) **EP 0 626 007 B1**
(45) Date of publication and mention of the grant of the patent: **02.06.1999**
(21) Application number: 93903340.3
(22) Date of filing: 20.01.1993
(51) Int. Cl.: C12M 1/12

(54) **CONTINUOUS MEMBRANE REACTOR AND USE THEREOF IN BIOTECHNICAL PROCESSES**
KONTINUIERLICHER MEMBRANREAKTOR UND SEINE ANWENDUNG IN BIOTECHNISCHEN PROZESSEN
REACTEUR A MEMBRANE A FONCTIONNEMENT CONTINU ET SON APPLICATION DANS LES PROCESSUS BIOTECHNIQUES

(30) Priority: 22.01.1992 NO 920289
(43) Date of publication of application: 30.11.1994
(73) Proprietor: NORSKE MEIERIER, N-0187 Oslo (NO)
(72) Inventor: LYSBERG, Magne, N-7078 Saupstad (NO); STORRO, Ivar, N-7033 Trondheim (NO); SVENDSEN, Hallvard, N-7035 Trondheim (NO); WINNBERG, Asgeir, N-7075 Tiller (NO); EIDE, Ola, N-0667 Oslo (NO); OTERHOLM, Bjarne, N-1430 As (NO)
(74) Representative: Onn, Thorsten
(86) International application number: NO9300015
(87) International publication number: WO9315182

(56) References cited:
- EP-A- 0 121 074
- EP-A- 0 224 800
- WO-A-85/00183
- US-A- 4 937 196
- Dialog Information Services, File 351, World Patent Index, Dialog Accession No. 007361140, WPI Accession No. 87-358146/51, TOSHIBA KK: "Fermentation system - comprises reactor divided by diaphragm into culture and feed liq. chambers and a filter membrane sepg. culture liq"; & JP,A,62 259 576, 11-11-87, 8751 (Basic).

## Description

The invention relates to a continuous membrane reactor for the production of biotechnical products such as concentrated solutions of anaerobic micro-organisms or facultative anaerobic micro-organisms. Other biotechnical products can also be produced in the apparatus, for instance protein hydrolysates produced by means of continuous protein aggregation.

The first industrial fermenting processes which were developed were based on the production of micro-organisms in reactors (fermenters) and on the concentration of the bacteria in separate concentration units. These first processes were operated batchwise. A batchwise process of this kind results in a low average volumetric productivity (increase in micro-organism quantity in relation to volume and time unit). In addition, due to the batchwise production, the load on the concentration unit from the micro-organism mass varied, so that this had to be overdimensioned in relation to the micro-organism production in the fermenter. The further development resulted in a continuous cultivation of micro-organisms in the fermenters. Volumetric productivity was thereby increased and the load on the concentration unit was more even. Productivity is dependent on the concentration of micro-organisms in the fermenter. With anaerobic growth the micro-organism concentration is limited through the inhibition of end products from the metabolism. Maximum micro-organism concentration is therefore limited by the growth of micro-organisms when the growth medium is optimalised. It is nevertheless possible to increase the micro-organism concentration beyond this limitation by connecting the fermenter to a concentration unit. In the concentration unit the inhibiting metabolism components are removed from the micro-organism mass. A portion of the produced micro-organism mass is returned to the fermenter and an "artificially" strong concentration of micro-organism mass is achieved in the fermenter. This is known technology and is used both in the production of ethanol and in biological purification of water.

The known prior art documents EP-A-0 121 074, WO-A-85/00l83 and EP-A-0 224 800 all refer to continuous membrane reactors with filter units incorporated in the reactor loop. None of them, however, discloses or even suggests the incorporation of a hydrocyclone in the reactor loop. Further, the prior equipment includes the use of a fermenter vessel or reactor, such as in EP-A-224 800, where the filtering apparatus is placed inside the bioreactor.

The invention's contribution to the development is that calculations showed that concentration units of the "cross-flow" type of filtering apparatus had a dead volume sufficiently large to be used as fermenter volume. The principal idea according to the invention is to grow micro-organisms and biotechnical products in the dead volume of the apparatus, so that the fermenter, which increases the cost of the process, is unnecessary.

The method according to the invention for continuous production of various biotechnical product groups such as anaerobic and facultative anaerobic micro-organism solutions, protein hydrolysates with an upper molecular weight limit and protein aggregates with a defined lower size, characterized in that the dead volume of the reactor loop and the filtering apparatus is used in the production. The invention also relates to the use of a continuous membrane reactor according to any one of the claims. The dead volume of the filtering apparatus may readily be changed by altering the dimension of the pipe coil, both in length and diameter. In order to achieve the same productivity with conventional batchwise fermentation, it is necessary to have a fermentor which is approx. 50 times larger than a filtering apparatus.

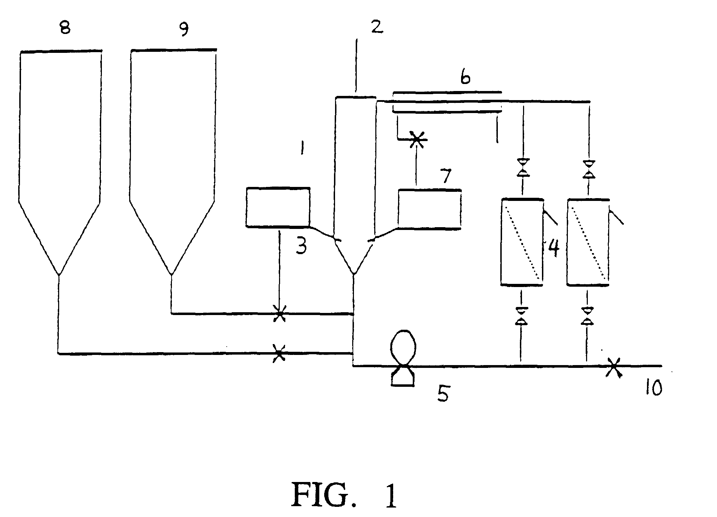

This type of high productivity reactor is shown in Figure 1.

Gasifying micro-organisms will create problems in such a closed system. The gas which is formed will not leave the system through the same filter as the spent growth medium. In order to cultivate gasifying micro-organisms, a hydrocyclon (1) is installed in the reactor loop in order to separate fluid and gas. The gas is removed through a separate gas filter (2) which can be sterilized. This gas must be removed from the micro-organism solution before the micro organisms are used for instance for a possible immobilization of hydro-colloidal gels. Nutrient medium and possibly other raw materials are added to the reactor loop from the raw material tank (8). The quantity of fluid in the hydrocyclone (1) is regulated through the relative speed between the filtrate flux and the pumping in of growth medium.

The pH of the reactor solution is measured (3) and regulated through the addition of chemicals from tank (9), which adjust the pH. The temperature in the reactor is regulated by means of a heat exchanger (6) which is controlled via a temperature measuring device (7).

Filtering of the reactor solution to remove spent medium or other reaction solutions takes place through cross-flow filtration in the filtering modules (4). All known filter types for ultra and micro filtration, produced both from inorganic and organic materials, may be used for filtration. The pore size of the filters are adapted to the requirements of the individual process. Circulation above the filter and through the hydrocyclone is brought about by a pump (5). A lobe pump is chosen particularly for this purpose due to its gentle operation which protects micro-organisms and protein particles. In order to achieve optimal filtration, the circulation speed and the in and out pressure of the filter unit, as well as the pressure on the filtrate side, are measured. Since the filter must be washed from time to time, the apparatus is equipped with two filter units (4), coupled in parallel, so that one is used for filtering of the reactor solution, while the other is cleaned and possibly changed and sterilized. Particles (micro-organisms and protein aggregates) and molecules (enzymes) which do not leave the filter reactor through the filtering system, can be drawn off separately (10),

The control of the rate of micro-organism growth is regulated through the addition of growth-inhibiting components.

In the following the invention will be explained by means of some examples which show some areas of application for the continuous membrane reactor.

### Example 1

The continuous membrane reactor is specially constructed for the production of anaerobic organisms, since the food industry mainly applies anaerobic starters, principally for the processing of milk, meat and vegetables.

The quality of the starter is determined by the growth conditions of the organisms in the production reactor. With a conventional production method the growth conditions are continuously changed during production. The quality of the starter thus varies with the time it is harvested, and with unforeseen variations in the medium during growth. One of the problems with traditional starter production is therefore to maintain correct quality of the micro-organisms.

The homo-fermentative lactic acid bacterium Lactobaccillus helveticus CNRZ 303 was grown in situ in a medium at 40°C and after 20 hours inoculated in a germ-free filtering equipment.

A sugar solution in the form of an ultra-filtered whey was poured into the plant (8). Inorganic nutrients are added to the whey as shown in table 1.

**TABLE 1**

| | |
|---|---|
| Addition of inorganic nutrients to ultra-filtered whey, fortified with yeast extract: | |
| MgSO₄7H₀ | 3 g |
| MnSO₄H₂O | 0.1 g |
| K₂HPO₄ | 4.4 g |
| FeSO₄/H₂ | 0.28 g |
| Whey | 1000 g |

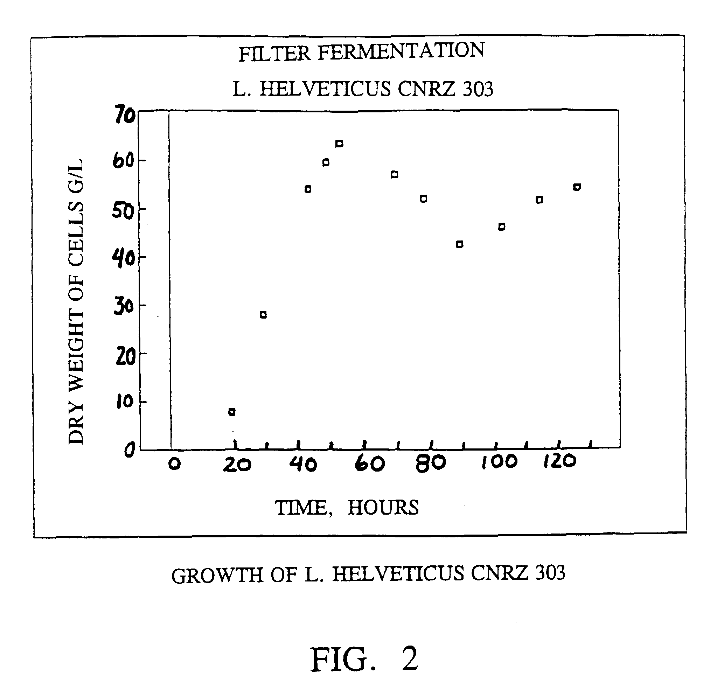

A solution of NaOH(9) is used as an acid neutralizer. Filtrate is pumped from the unit. A typical growth sequence is shown in Figure 2:

A concentrated micro-organism solution is pumped from the apparatus according to a plan when the micro-organism concentration has reached the desired level, for instance 60 g dry weight micro-organisms per litre (10).

Tests have confirmed that a continuous production of starters in the described membrane reactor is suitable for securing a correct and reproducible product quality. In the production phase all process parametres, including medium components and micro-organism concentration are constant over time, through controlled addition of growth-inhibiting components.

### Example 2

Available literature shows that milk allergy almost always occurs in small children in the age group 0-4 years. It has further been shown that milk products where the milk proteins have been reduced in size to under 10,000 D do not produce allergic reactions. Tests have been carried out for the production of milk which will not produce such allergy in exposed groups.

The purpose of the tests was to hydrolyse milk proteins to under 10,000 D by using commercial proteases or the fungus Mucor mucedo, a typical Norwegian fungus which has a long tradition in the production of limburger cheese, where the protease activity of this fungus is used for hydrolysis of proteins in skimmilk. The relevant production process is generally dependent upon a reactor where the environmental conditions for optimal protease activity can be controlled (temperature, pH, mineral concentration, etc.). During the process it will also be necessary to continuously separate proteins with a size under 10,000 D from the larger milk proteins and the hydrolysed enzymes. The continuous membrane reactor according to the invention contains the technical solutions to the conditions mentioned and therefore seems ideal for this purpose. In the membrane reactor there is achieved a reduced consumption of enzyme, in that the enzyme is retained in the reactor loop (active immmobilization of the enzyme). Neither do the enzymes follow the product stream and thereby they cannot cause uncontrolled protein hydrolysis and the formation of bitter components outside the membrane reactor. In addition it has been assured that the milk product does not receive an addition of protein (enzyme) with a molecular weight of more than 10,000 D which may produce allergies.

### Example 3

Dairy products with a reduced fat content have had a rapid success in the market. This particularly applies to milk as a drink, where 2 per cent milk is the leading product. Other types of dairy products with reduced fat content, for instance cheese and yogurt, are generally considered by the consumers to be of poorer quality than ordinary products. The reason is mainly linked to factors such as texture and taste.

Market trends still indicate that people more and more will switch from ordinary products to products with a reduced fat content, and prognoses point to an increase in this trend in the near term.

Since protein particles with a size in the order of 0.1-3 µm may have characteristics which resemble fat, such protein-based fat substitutes are today in demand as components for quality improvement of foodstuffs with a reduced fat content.

The production and concentration of such protein particles with the given size requires strict control of kinetic and environmental conditions (temperature, pH and mineral composition). The continuous membrane reactor according to the invention will provide good control of these parametres.

## Claims

1. An apparatus for the continuous production of biotechnical substances in a nutritive medium, **characterized** in that said apparatus does not comprise a fermenter and consists of a hydrocyclone (1), filter means (4), connective conduits, means (5) for keeping the medium in circulation through said hydrocyclone, filter means and conduits, and means (3, 7) for measuring and influencing pH and temperature.

2. Apparatus according to claim 1, **characterized** in that said apparatus comprises at least two filter modules, arranged in parallel.

3. Apparatus according to claim 1, **characterized** in that said means for keeping the medium in circulation is a pump.

4. Apparatus according to claim 1, **characterized** in that said means for measuring and influencing pH and temperature comprise a pH monitoring device (3), operatively connected to a device (9) for the addition of chemicals which adjust the pH and a temperature measuring device (7), operatively connected to a heat-exchanger (6), through which the circulating medium passes.

5. A method for the production of biotechnical substances using an apparatus according to any one of claim 1-4, **characterized** in that the dead volume of the filter/s and conduits in which the medium circulates in said apparatus is used for the production of a biotechnical substance.

6. A method according to claim 5, **characterized** in that control of the rate of micro-organism growth is regulated through the addition of growth-inhibiting components.

7. A method according to any one of claim 5-6, **characterized** in that Mucor mucedo or enzymes thereof are used in the production of protein hydrolysates.

## Patentansprüche

1. Apparat zur kontinuierlichen Herstellung biotechnologischer Substanzen in einem Nährmedium, dadurch gekennzeichnet, daß dieser Apparat keinen Fermentierer umfaßt und aus einem Hydrozyklon (1), Filtervorrichtungen (4), Verbindungs-leitungen, einer Einrichtung (5) zum Zirkulieren des Mediums durch den Hydrozyklon, Filtereinrichtunyen und Leitungen, und Vorrichtungen (3,7) zum Messen und Beeinflussen von pH-Wert und Temperatur besteht.

2. Apparat nach Anspruch 1, dadurch gekennzeichnet, daß der Apparat mindestens zwei Filtermodule in paralleler Anordnung umfaßt.

3. Apparat nach Anspruch 1, dadurch gekennzeichnet, daß die Einrichtung zum Zirkulieren des Mediums eine Pumpe ist.

4. Apparat nach Anspruch 1, dadurch gekennzeichnet, daß die Vorrichtung zum Messen und Beeinflussen von pH-Wert und Temperatur eine pH-Überwachungsvorrichtung (3) umfaßt, die betriebsmäßig an eine Vorrichtung (9) zur Zugabe einer Chemikalie zur Einstellung des pH-Wertes und eine Temperatur-Meßgerät (7) angeschlossen ist, das betriebsmäßig mit einem Wärmeaustauscher (6) verbunden ist, durch den das zirkulierende Medium wandert.

5. Verfahren zur Herstellung biotechnologischer Substanzen unter Verwendung eines Apparates nach einem der Ansprüche 1-4 dadurch gekennzeichnet, daß das Totvolumen des/der Filter/s und Leitungen, in denen das Medium in diesem Apparat zirkuliert, zur Herstellung einer biotechnologischen Substanz verwendet wird.

6. Verfahren nach Anspruch 5, dadurch gekennzeichnet, daß die Kontrolle der Rate des Mikroorganismen-Wachstums durch die Zugabe wachstumshemmender Komponenten reguliert wird.

7. Verfahren nach einem der Ansprüche 5-8, dadurch gekennzeichnet, daß Mucor mucedo oder Enzyme davon bei der Herstellung von Protein-Hydrolysaten verwendet wird.

## Revendications

1. Appareil pour la production continue de substances biotechniques dans un milieu nutritif, caractérisé en ce que ledit appareil ne comprend pas de fermenteur et se compose d'un hydrocyclone (1), de moyens de filtration (4), de conduits de raccordement, de moyens (5) pour maintenir le milieu en circulation à travers ledit hydrocyclone, lesdits moyens de filtration et lesdits conduits, et de moyens (3, 7) pour mesurer et modifier le pH et la température.

2. Appareil selon la revendication 1, caractérisé en ce que ledit appareil comprend au moins deux modules de filtration, montés en parallèle.

3. Appareil selon la revendication 1, caractérisé en ce que lesdits moyens pour maintenir le milieu en circulation sont constitués par une pompe.

4. Appareil selon la revendication 1, caractérisé en ce que lesdits moyens pour mesurer et modifier le pH et la température comprennent un dispositif de contrôle de pH (3) raccordé en service à un dispositif (9) pour l'adjonction de produits chimiques qui permettent le réglage du pH, et un dispositif de mesure de température (7), raccordé en service à un échangeur de chaleur (6), à travers lequel le milieu en circulation passe.

5. Procédé pour la production de substances biotechniques à l'aide d'un appareil selon l'une quelconque des revendications 1 à 4, caractérisé en ce que le volume mort du ou des filtre(s) et des conduits, dans lesquels le milieu circule dans ledit appareil, est utilisé pour la production d'une substance biotechnique.

6. Procédé selon la revendication 5, caractérisé en ce que le contrôle de la vitesse de croissance des micro-organismes est régulé par l'adjonction de composés inhibiteurs de croissance.

7. Procédé selon l'une quelconque des revendications 5 et 6, caractérisé en ce que le Mucor mucedo ou ses enzymes sont utilisés lors de la production d'hydrolysats de protéines.
